Europäisches Patentamt

European Patent Office

Office européen des brevets

(19)

(11) Publication number: **0 221 653**
**A2**

(12) **EUROPEAN PATENT APPLICATION**

(21) Application number: **86307098.3**

(22) Date of filing: **15.09.86**

(51) Int. Cl.⁴: **A 61 K 49/02**

(30) Priority: **20.09.85 US 778591**

(43) Date of publication of application:
**13.05.87 Bulletin 87/20**

(84) Designated Contracting States:
**AT BE CH DE FR GB IT LI LU NL SE**

(71) Applicant: **THE REGENTS OF THE UNIVERSITY OF CALIFORNIA**
**2199 Addison Street**
**Berkeley California 94720(US)**

(72) Inventor: **Engelstad, Barry L.**
**61 El Toyonal**
**Orinda California 94563(US)**

(72) Inventor: **O'Connell, John William**
**812 "1" Street**
**Petaluma California 94952(US)**

(74) Representative: **Bizley, Richard Edward**
**BOULT, WADE & TENNANT 27 Furnival Street**
**London EC4A 1PQ(GB)**

(54) **Method of dual isotope color functional imaging utilizing radiolabelled monoclonal antibodies, kits therefor and antibodies for use therein.**

(57) A dual isotope imaging method is provided whereby a dual isotope color functional image is produced to detect targeted tissued by color. Kits and antibodies for such a method and the provision of such antibodies are also included.

EP 0 221 653 A2

## METHOD OF DUAL ISOTOPE COLOR FUNCTIONAL IMAGING UTILIZING RADIOLABELLED MONOCLONAL ANTIBODIES, KITS THEREFOR AND ANTIBODIES FOR USE THEREIN

The present invention is directed to a method of in vivo imaging of tissues within a living subject, and to kits therefor and antibodies for use therein. In particular, the present invention is directed to a method of imaging target tissues within a living subject utilizing radiolabelled monoclonal antibodies in dual isotope color functional imaging.

Radioimmunoimaging has been utilized as a diagnostic medical procedure whereby one or more radiolabelled immunoglobulins are administered to a living subject and the resultant distribution of the labelled immunoglobulin is imaged, for example, by scintigraphy to detect and localize abnormal tissue, such as a tumor. Dual isotope imaging is an example of this technique whereby two different radiolabelled immunoglobulins are administered to the subject, one of the immunoglobulins being reactive with tumor-associated antigens and the other being nonreactive with tumor-associated antigens. The two immunoglobulins are radiolabelled with isotopes so as to permit differentiation in the imaging process based on energies of emission. The purpose of the dual isotope technique is to attempt to deal with the problem of "background" localization when detecting the signal of the radiolabelled immunoglobulin which is reactive with the tumor-associated antigens. Ideally, the immunoglobulin, which is a monoclonal antibody, will be specific for the antigens at the site of the target tissue, such as a tumor, and therefore initially one would expect that there would be no problem with "background" localization. That is, one would expect that all or a substantial portion of the radiolabelled immunoglobulin would localize at the site of the tumor and that very little, if any, of the immunoglobulin would be associated with nontarget tissue. However, in actual practice, much of the immunoglobulin specific for the tumor-associated antigens is not found at the target tumor, but is found elsewhere in the body. Thus, a typical scintigram of a single radiolabelled monoclonal antibody specific for a tumor will reveal a distribution of the isotope in various regions of the subject, leaving it to the operator to surmise that the region or regions of highest intensity (i.e., highest concentration of the radioisotope) correspond to tumor material. In some cases, the region or regions of highest intensity may not necessarily correspond to the tumor, and in cases where there are more than one region of highest intensity, one cannot necessarily distinguish tumor material from nontumor material.

Therefore, the concept of dual isotope imaging deals with the problem of "background" localization. The second radiolabelled immunoglobulin is selected to be nonreactive with tumor-associated antigens. It is therefore presumed that the highest concentration of the second radiolabelled immunoglobulin will not occur at the tumor. Therefore, by subtraction of the signals of the first labelled immunoglobulin in locations where there is also a signal due to the presence of the second radiolabelled immunoglobulin, it might be expected that the resultant image of the first radiolabelled immunoglobulin would show only the areas where the tumors exist. However, in practice, this is not necessarily the case and there are several disadvantages to dual isotope imaging using this subtraction technique.

One of the disadvantages is that when using dual isotope imaging where the image of one isotope is used to subtract background from the other, information is obviously being deleted from the display. This information may be valuable to the diagnostician, for example, in assessing the relative uptake of various tissues of the immunoglobulin. Thus, by using the subtraction method, only the tissues containing the highest concentration (i.e., above a preselected threshold level) will appear on the image.

Another disadvantage of dual isotope imaging using the subtraction method is that one is unable to consistently assign the likelihood, continuously over time, of the location of the target tissue.

The present invention provides a method of dual isotope imaging without the above disadvantages.

The present invention is thus based upon a method of dual isotope in vivo imaging by the relative uptake by target and nontarget tissues of the radiolabelled monoclonal antibodies which can be monitored in a continuous fashion. Ideally, there is provided a method of dual isotope imaging whereby the correct assignment by probability of the location of target tissues can be performed directly from color functional images.

The present invention provides a method for in vivo imaging of tissues in a living subject, comprising the steps of administering to the subject a first radiolabelled monoclonal antibody or fragment thereof and a second radiolabelled monoclonal antibody, or fragment thereof, wherein the first monoclonal antibody is specifically reactive with one or more antigens associated with a target tissue and the second monoclonal antibody is nonreactive with antigens associated with the target tissue, but otherwise similar. The respective radiolabels on the first and second monoclonal antibodies are different from each other.

Thus, the invention provides in one aspect a kit for use in tissue imaging is a subject comprising a frist radiolabelled monoclonal antibody, or fragment thereof, and a second radiolabelled monoclonal antibody, or fragment thereof, said first monoclonal antibody being specifically reactive with one or more antigens associated with a target tissue; and said second monoclonal antibody being nonreactive with antigens associated with said target tissue; respective radiolabels on said first and second monoclonal antibodies being different from each other; said antibodies after administration to the subject being adapted to form a colored dual isotope functional image, said functional image derived from a selected energy of emission of said first radiolabel and a selected energy of emission of said second-radiolabel to form an intensity weighted color image and the relative concentrations and location of said radiolabels; and the difference between respective energies of selected photopeaks being of a magnitude sufficient to avoid crosstalk between said photopeaks in the formation of respective monochromatic images.

The observer can correctly assign by probability the location of the target tissues by forming a colored dual isotope functional image derived from a selected energy of emission of that radiolabelled monoclonal antibody, and a selected energy of emission of the second radiolabelled monoclonal antibody. The differences between the respective energies of emission of the selected photopeaks of the two radioisotopes is selected such that it is of a magnitude sufficient to avoid crosstalk between the two observed photopeaks used in the formation of the respective monochromatic images. Crosstalk is interference caused by the coupling of energy by one of the measured photopeaks into the other measured photopeak. The data from these two energy levels is used to form an intensity weighted color image of the relative concentrations and location of the two radiolabelled monoclonal antibodies.

In the accompanying FIGURES:

FIG. 1 is a set of scintigrams (1 day, 2 days, 7 days post-injection) according to the present invention showing two monochromatic images and a composite thereof in a mouse after the injection of Ga-67 and In-111 labelled monoclonal antibodies.

FIG. 2 is a set of scintigram (1 day, 2 days, 7 days post-injection) according to the present invention showing a composite colored image of a mouse after injection of Ga-67 and In-111 labelled monoclonal antibodies.

The present invention relies upon a method of in vivo imaging tissues utilizing two radiolabelled monoclonal antibodies. Broadly, the method according to the present invention requires a first monoclonal antibody, or immunoglobulin, of a type which is specifically reactive with antigens at the tissue to be targeted, and a second monoclonal antibody, or immunoglobulin, which is nonreactive with antigens at the site to be targeted. In the usual case, the tissue to be targeted will be a tumor or comprise tumor cells. Methods of producing monoclonal antibodies which are specific against tumor-associated antigens are known, as are monoclonal antibodies which are nonreactive with tumor-associated antigens. A matched subclass pair of monoclonal antibodies may be utilized, provided one of such antibodies is specific for the tumor antigens and the other monoclonal antibody is nonspecific for tumor antigens. For example, the first monoclonal antibody may be selected from antimelanoma monoclonal antibodies, such as XOMA XMMME 0002, and the second monoclonal antibody, not reactive with melanoma cells, may be selected from antibodies not reactive with any antigens in the body, for example, the immunoglobulin XOMA IC10.

The monoclonal antibodies may be labelled with a radioactive isotope according to known methods. A preferred method is that disclosed by Hnatowich, et al., Science 220: 613-615 (1983), using the cyclic anhydride of DTPA. Other agents for linking a protein to a radiometal may be used, such as desferrioxamine B.

The radioactive labels utilized to label the monoclonal antibodies may be any of those isotopes known in isotope imaging in general. These include radioactive isotopes of indium, gallium, and technetium. Technically preferred are the isotopes In-111 and Ga-67. Other isotopes include Tc-99M, and In-113M.

The labelled monoclonal antibodies may be administered to the subject according to any appropriate method, which will depend at least in part on the target tissue and its suspected location in the subject. In the usual case, the labelled monoclonal antibodies will be administered intravenously. The amount to be administered of each labelled monoclonal antibody will depend on the size of the subject, the nature of the tissue which is targeted, the sensitivity of the instrument which is to detect the radioemissions, and the like, and therefore will be adjusted to be optimized within these parameters. It is preferred that the two labelled monoclonal antibodies be administered to the subject simultaneously, however, this is not a requirement. When administered simultaneously, the gradual uptake of the respective labelled monoclonal antibodies in the various tissues in the body may be followed over time in accordance with the present invention and may give useful information with regard to the proper functioning of systems and organs of the subject.

According to the present invention, each of the radioisotopes will be detected by radioemission and a selected energy of emission. The particular photopeaks to be monitored must have energies sufficiently different so as not to cause crosstalk, thereby avoiding false signals. In the case of the preferred indium 111 isotope, the photopeak at 247 keV will be utilized when the other radioisotope is gallium 67. The photopeak of gallium 67 to be monitored will be that at 93 keV. The energy separation between these two photopeaks is sufficient to minimize crosstalk and maximize counting efficiency.

Data from the emissions from the two radioisotopes will be processed into color parametric images which encode in hue the relative activity of the detected radioisotope as well as intensity. This method has been disclosed by O'Connell, et al., "Color Composites: Display Of Two Independent Parameters In A Single Functional Image", Emission Computed Tomography: Current Trends, P. Esser, ed., Society of Nuclear Medicine, Inc., N.Y., 275-287 (1983). The instruments for collecting, processing and displaying the data as parametric color images are available such as from Digital Equipment (DEC-PDP-11 data processor), ADAC Labs, or Computer Design and Applications (Delta-11 display). The dual isotope functional image is formed in the following way. Each picture element (pixel) has a value in the 247 keV (indium 111) image and a value in the 93 keV (gallium 67) image. Together these values give the Cartesian coordinates of a vector, or a point in the plane. This vector can also be represented in polar coordinates as a magnitude and an angle. Polar magnitude is a composite of the absolute intensity levels in the two images, while polar angle is a measure of the relative contribution of each image to the composite. The color image maps magnitude to intensity and angle to hue, using an IHS (Intensity, Hue, Saturation) model of color space. Saturation is kept maximal. The color image thus provides an intensity weighted color map of the relative concentrations of the two isotopes, which corresponds to a map giving the probability of existence of target tissue at any location. In the foregoing instance, the area having the highest probability of being the target tissue will thus appear in the dual isotope functional image as a red area, indicating a virtually total presence of indium 111 in the absence of any gallium 67. Areas of lower concentration of indium 111 will be in various hues of orange, yellow, green, blue and gray.

It will be appreciated that not only monoclonal antibodies but also monoclonal antibody fragments, such as the Fab or F(ab')2, may be used instead of the full immunoglobulins. Mixtures, so called cocktails, of reactive or nonreactive monoclonal antibody products may be used to broaden the spectrum of applicability within the patient within a subject population.

The method of image processing described above may also be modified within the scope of the present invention. For example, the dual parameter color image may encode other calculated parameters such as hue which may be programmed to encode relative activity while intensity at each hue encodes the absolute difference in counts.

The uses of the method according to the present invention include imaging of specific tissues, such as tumors, or other abnormal tissues. In addition, the present invention may be utilized to follow the dynamics of uptake of the administered reagents over the course of time.

In order to describe the invention, the following example is provided by way of illustration but is not intended to limit the invention in any way.

<u>Example</u>

Antimelanoma monoclonal antibodies (XOMA-XMMME0002) were radiolabelled with indium 111 using cyclic DTPA dianhydride, in accordance with the method disclosed in Hnatowich, <u>et al</u>., <u>supra</u>. The control monoclonal antibodies (<u>i.e.</u>, not reactive <u>in vitro</u> with melanoma cells) of the same immunoglobulin subclass (XOMA IC10) were radiolabelled in parallel using the same method except for the radionuclide, gallium 67. The products (150uCi in 6 ug

XMMME0002-DTPA-111-indium; 300 uCi in 12 ug IC10-DTPA-67-gallium) were administered intravenously simultaneously into 6 athymic mice bearing human melanomas (XOMA Minor cell line, approximate size 0.5 centimeters; right-hand flank). Scintigrams were obtained at 1,2 and 7 days post-injection using a gamma camera (pinhole collimator). The data was digitally stored on computer in two image matrices. One matrix stored counts obtained in a 20% window symetrically set about the 93 keV principal photopeak of Ga-67. The other matrix stored counts obtained in a 20% window symetrically set about the 247 keV principal photopeak of indium-111. The standards of each isotope were imaged to check if any crosstalk had occurred. Color parametric images were then produced according to the method of O'Connell (supra) on a Digital Equipment PDP-11 data processor with a Delta-11 display (Computer Design and Application) that encoded in hue the relative activity while encoding in intensity the absolute activity. The resultant images permit a unique identification of tumors by their red, intense appearance.

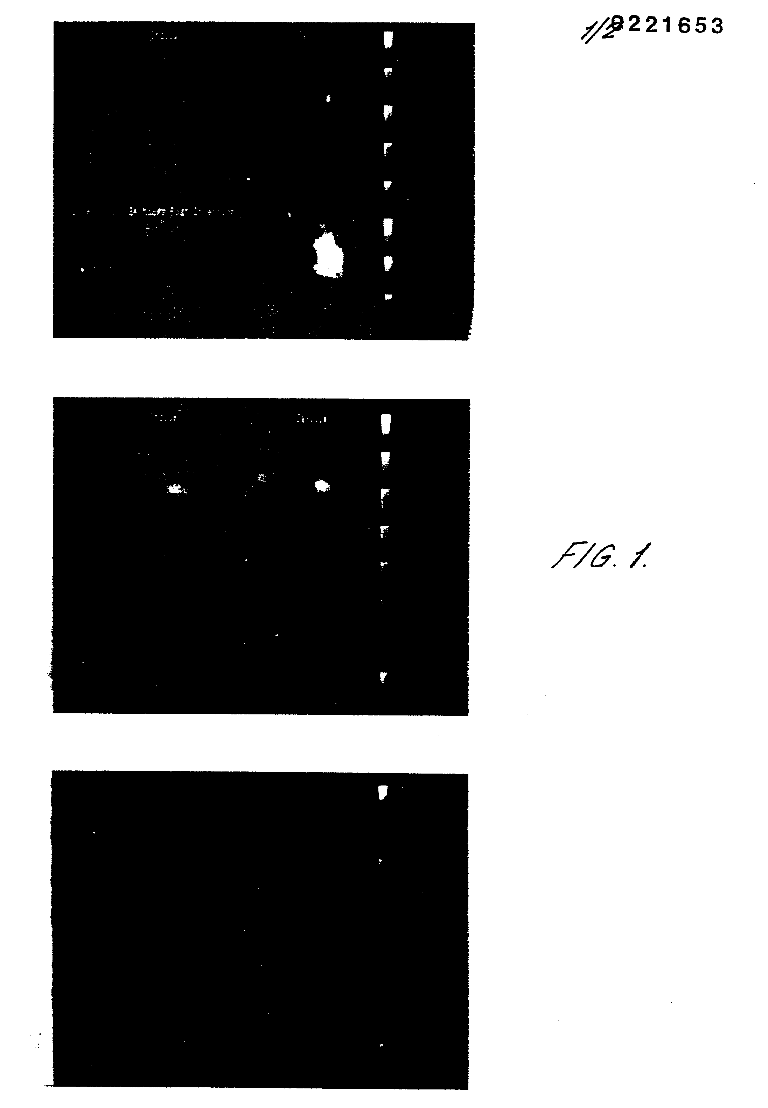

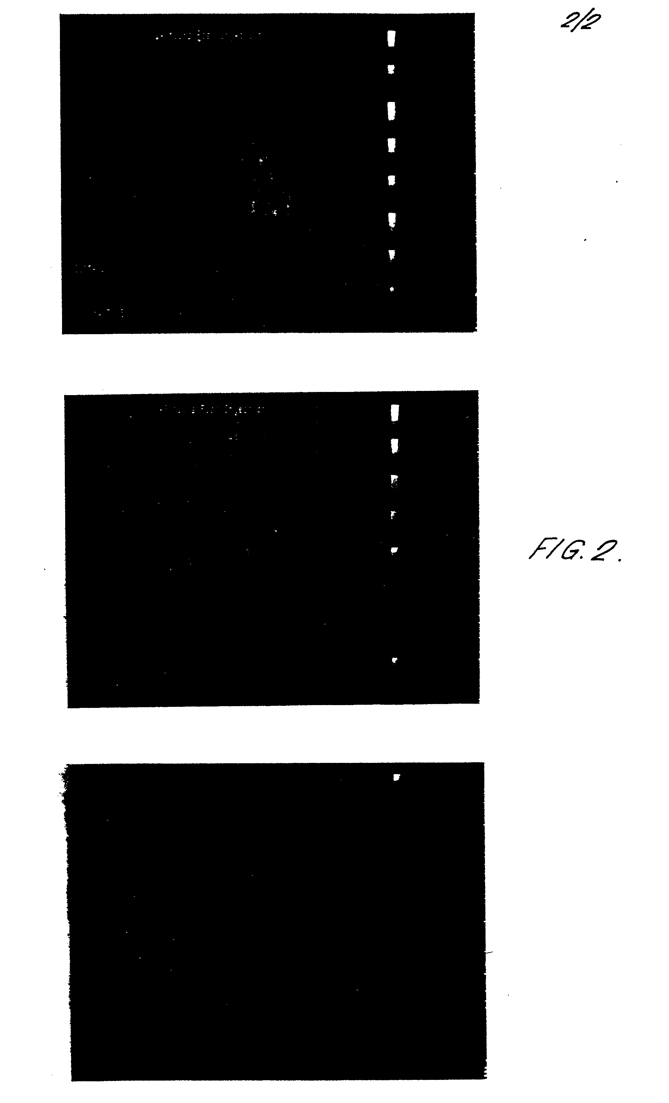

In FIG. 1 there is shown in mouse No. 6 after one week three separate images. In the upper left-hand corner of the figure is shown the red image due only to the indium-111; in the upper right-hand corner is shown the blue image due only to the gallium-67; in the lower right-hand is shown the composite image of the two images being superimposed. The color scale on the right shows the colors of relative probability of the location of targeted tumor, with red being the most probable location. Referring to FIG. 2, there is shown the combined dual color image in mouse No. 1 after one week. Again, as shown in FIG. 2, the red color appears in the right-hand flank of the mouse.

CLAIMS:

1.  A method of _in vivo_ imaging of tissues in a subject, comprising the steps of administering to said subject the first radiolabelled monoclonal antibody, or fragment thereof, and a second radiolabelled monoclonal antibody, or fragment thereof, said first monoclonal antibody being specifically reactive with one or more antigens associated with a target tissue; and said second monoclonal being nonreactive with antigens associated with said target tissue; respective radiolabels on said first and second monoclonal antibodies being different from each other; and forming a colored dual isotope functional image, said functional image derived from a selected energy of emission of said first radiolabel and a selected energy of emission of said second-radiolabel to form an intensity weighted color image and the relative concentrations and location of said radiolabels; wherein the difference between respective energies of selected photopeaks is of a magnitude sufficient to avoid crosstalk between said photopeaks in the formation of respective monochromatic images.

2.  A method according to Claim 1 wherein said first monoclonal antibody or fragment thereof is selectively reacted with tumor-associated antigens and second monoclonal antibody or fragment thereof is nonreactive with the tumor-associated antigens.

3.  A method according to Claim 1 or Claim 2 wherein said first and second radiolabels are independently selected from the group consisting of radioisotopes of indium, gallium and technetium.

4.  A method according to Claim 3 wherein said first radiolabel comprises indium-111 and said second radiolabel comprises gallium-67.

5. A method according to Claim 4 wherein the selected photopeak of indium-III is at 247 keV and the selected photopeak of gallium-67 is at 93 keV.

6. A kit for use in tissue imaging in a subject comprising a first radiolabelled monoclonal antibody, or fragment thereof, and a second radiolabelled monoclonal antibody, or fragment thereof, said first monoclonal antibody being specifically reactive with one or more antigens associated with a target tissue; and said second monoclonal antibody being nonreactive with antigens associated with said target tissue; respective radiolabels on said first and second monoclonal antibodies being different from each other; said antibodies after administration to the subject being adapted to form a colored dual isotope functional image, said functional image derived from a selected energy of emission of said first radiolabel and a selected energy of emission of said second radiolabel to form an intensity weighted color image and the relative concentrations and location of said radiolabels; and the difference between respective energies of selected photopeaks being of a magnitude sufficient to avoid crosstalk between said photopeaks in the formation of respective monochromatic images.

7. A radiolabelled monoclonal antibody for use in a method as defined in Claim 1.

8. A method for providing reagents for use in tissue imaging in a subject, which method comprises selecting a first radiolabelled monoclonal antibody, or fragment thereof, and a second radiolabelled monoclonal antibody, or fragment thereof, said first monoclonal antibody being specifically reactive with one or more antigens associated with a target tissue; and said second monoclonal antibody being nonreactive with antigens associated with said target tissue; respective radiolabels on said first and second monoclonal antibodies being different from each other; said antibodies after administration to the subject being adapted to form a colored dual isotope functional image, said functional image derived from a selected energy of emission of said first radiolabel and a selected energy of emission of said second radiolabel to form an intensity weighted color image and the relative concentrations and location of said radiolabels; and said antibodies also being chosen such that the difference between respective energies of selected photopeaks is of a magnitude sufficient to avoid crosstalk between said photopeaks in the formation of respective monochromatic images.

9.     The use of radiolabelled monoclonal antibodies in providing reagents for use in tissue imaging in a subject by selecting a first radiolabelled monoclonal antibody, or fragment thereof, and a second radiolabelled monoclonal antibody, or fragment thereof, said first monoclonal antibody being specifically reactive with one or more antigens associated with a target tissue; and said second monoclonal antibody being nonreactive with antigens associated with said target tissue; respective radiolabels on said first and second monoclonal antibodies being different from each other; said antibodies after administration to the subject being adapted to form a colored dual isotope functional image, said functional image derived from a selected energy of emission of said first radiolabel and a selected energy of emission of said second radiolabel to form an intensity weighted color image and the relative concentrations and location of said radiolabels; and said antibodies also being chosen such that the difference between respective energies of selected photopeaks is of a magnitude sufficient to avoid crosstalk between said photopeaks in the formation of respective monochromatic images.

10. A kit as claimed in Claim 6, an antibody as claimed in Claim 7, a method as claimed in Claim 8, or the use claimed in Claim 9, in each case further defined by the specific features(s) of any one or more of Claims 2 to 5.

FIG. 1.

FIG.2.